# EUROPÄISCHE PATENTSCHRIFT

(11) **EP 2 406 803 B1**
(45) Veröffentlichungstag und Bekanntmachung des Hinweises auf die Patenterteilung: **11.05.2016**
(21) Anmeldenummer: 10709400.5
(22) Anmeldetag: 04.03.2010
(51) Int. Cl.: H01H 13/06, H01H 13/48

(54) **TASTER, VERFAHREN ZUR HERSTELLUNG EINES SOLCHEN UND MEDIZINISCHES HANDHABUNGSTEIL**
PUSH BUTTON, METHOD FOR THE PRODUCTION THEREOF, AND MEDICAL MANIPULATING PART
BOUTON-POUSSOIR, SON PROCÉDÉ DE FABRICATION ET MANCHE D'INSTRUMENT MÉDICAL

(30) Priorität: 12.03.2009 DE 102009012911; 26.05.2009 DE 102009022687
(43) Veröffentlichungstag der Anmeldung: 18.01.2012
(73) Patentinhaber: ERBE Elektromedizin GmbH, 72072 Tübingen (DE)
(72) Erfinder: SCHNITZLER, Uwe, 72074 Tübingen (DE); HAGG, Martin, 72827 Wannweil (DE); BELLER, Jürgen, 72810 Gomaringen (DE)
(74) Vertreter: Rüger, Barthelt & Abel
(86) Internationale Anmeldenummer: PCT/EP2010/001365
(87) Internationale Veröffentlichungsnummer: WO 2010/102754

(56) Entgegenhaltungen:
- EP-A2- 0 323 917
- DE-A1- 3 713 318
- GB-A- 1 195 702
- GB-A- 1 441 008
- US-A- 4 121 070
- US-A- 5 294 241

## Beschreibung

Die Erfindung betrifft einen Taster, insbesondere als Teil eines medizinischen Handhabungsteils, wie etwa eines medizinischen Instrumentes oder eines Handgriffs hiervon. Sie betrifft des Weiteren ein Verfahren zur Herstellung eines solchen Tasters sowie ein medizinisches Handhabungsteil.

Mit dem zunehmenden Einsatz der HF-Chirurgie einerseits und von elektrisch betriebenen oder betätigten Diagnose- und Therapieinstrumenten andererseits hat sich der Einsatz von handgeführten medizinischen Instrumenten bzw. Geräten stark ausgeweitet, deren Handhabung die Auslösung von Schaltfunktionen umfasst. Bei derartigen Instrumenten und Geräten bzw. den zugehörigen Handhabungsteilen werden elektrische Tastschalter (nachfolgend bezeichnet als "Taster") eingesetzt.

Es besteht hier das Problem, dass durch das Eindringen von Flüssigkeiten, durch die Anwendung, aber auch durch die Reinigung, Desinfektion und Sterilisation der Produkte die verwendeten Taster dahingehend Schaden nehmen können, dass Feuchtigkeit in diese eindringt und einen Ausfall der Taster/Kontakte bewirkt, wodurch die Funktionalität des Produktes nicht mehr gewährleistet ist.

Diese Art von Handhabungsstellen werden an den Schnittstellen wie Kabelaustritt, Kontaktsteckbereich, Tastenbereich dahingehend abgedichtet (mittels Dichtringen, Folien, Verkleben etc.) dass ein Eindringen von Flüssigkeiten vermieden wird, um die Tastelemente und deren Funktion zu schützen. Zusätzlich sind die Taster spritzwasserdicht ausgeführt, allerdings bietet dies aufgrund der Umgebungsbedingungen hinsichtlich der Sterilisation mittels feuchter Hitze nur einen bedingten Schutz.

Ein Taster mit den Merkmalen des Oberbegriffs von Anspruch 1 ist aus der US 4 121 070 A bekannt.

Mit dem vermehrten Einsatz von Handhabungsteilen der genannten Art haben sich bestimmte Probleme gezeigt. Zum einen ist ein erheblicher konstruktiver Aufwand nötig, um sämtliche Schnittstellen der Handhabungselemente abzudichten, zum Anderen besteht das Risiko einer Undichtheit nach mehrfacher Aufbereitung. Tritt an einer Stelle Feuchtigkeit ein, sind die anderen Abdichtungen ohne Funktion, sodass Feuchtigkeit ins Innere der Griffe gelangen kann.

Der Erfindung liegt daher die Aufgabe zugrunde, einen im Hinblick auf die o.g. Einsatzanforderungen verbesserten Taster anzugeben, der insbesondere eine große Lebensdauer und hohe Zuverlässigkeit aufweisen soll. Es soll des Weiteren ein entsprechend verbessertes Handhabungsteil bereitgestellt werden.

Diese Aufgabe wird durch einen Taster mit den Merkmalen des Anspruchs 1 bzw. durch ein Handhabungsteil mit den Merkmalen des Anspruchs 5 gelöst. Des Weiteren wird ein Verfahren zur Herstellung eines verbesserten Tasters der genannten Art bereitgestellt, welches die Merkmale des Anspruchs 9 aufweist. Zweckmäßige Fortbildungen des Erfindungsgedankens sind Gegenstand der jeweiligen abhängigen Ansprüche.

Die Erfindung schließt den Gedanken ein, einen ohne zusätzliche Dichtmittel (die latent mängelbehaftet sind) auskommenden Taster anzugeben, der eine minimale Anzahl von gegeneinander allein aufgrund ihrer Konstruktion hermetisch abgedichteten Teilen aufweist. Sie schließt weiter den Gedanken ein, eine Schaltfeder des Tasters als integralen Abschnitt eines seiner Gehäuseteile auszubilden, sodass eine Abdichtung zwischen jenem Gehäuseteil und der Schaltfeder von vornherein entfällt. Weiter schließt die Erfindung den Gedanken ein, neben jenem ersten Gehäuseteil zur Bildung des Gehäuses lediglich noch ein zweites Gehäuseteil vorzusehen, welches mindestens einen Kontakt des Tasters enthält. Schließlich gehört zur Erfindung der Gedanke, jenen Kontakt bzw. jene Kontakte in das zweite Gehäuseteil ohne separate Dichtmittel hermetisch dicht einzubetten.

Taster der erfindungsgemäßen Art sind besonders vorteilhaft einsetzbar in medizinischen Handhabungsteilen, daneben aber auch in anderen Geräten und speziell solchen, die hohen thermischen bzw. klimatischen Beanspruchungen ausgesetzt sein können.

Unter "hermetisch dicht" wird hier ein den üblichen Anforderungen bei der Sterilisation (insbesondere Dampfsterilisation) medizinischer Instrumente sowie bei deren Einsatz genügender Grad an Feuchtigkeits- und Druckdichtigkeit, auch bei thermischer Schock- und Wechselbelastung, verstanden. Hochvakuumdichtigkeit ist hierbei ebenso wenig gefordert wie Dichtigkeit bei extrem tiefen Temperaturen.

In einer Ausführung der Erfindung ist vorgesehen, dass das zweite Gehäuseteil mindestens abschnittsweise aus Glas besteht und der Gegenkontakt in den Glasabschnitt eingeschmolzen ist. Das Einschmelzen eines metallischen Teiles in Glas ist eine aus der Lichttechnik seit Jahrzehnten bekannte Technik, um hermetische Abdichtungen auf einfache, kostengünstige und über eine lange Lebensdauer zuverlässige Weise zu realisieren.

In einer weiteren Ausführung ist vorgesehen, dass das erste Gehäuseteil aus federelastischem Metall, insbesondere Edelstahl oder Titan oder einer Titanlegierung, besteht und insbesondere als Tiefziehteil ausgeführt ist. Hierdurch lässt sich die integrale Ausführung der Schaltfeder mit dem ersten Gehäuseteil ebenso leicht realisieren wie eine dauerhafte Korrosionsbeständigkeit und Dichtigkeit des Tasters auch unter den anspruchsvollen Bedingungen medizinischer Sterilisationsverfahren.

In einer Kombination der beiden genannten Ausführungen bietet sich eine Ausgestaltung als vorteilhaft an, bei der das zweite Gehäuseteil vollständig aus Glas besteht und sein die Wand des ersten Gehäuseteils berührender Umfang mit jener Wand eine physikalisch-chemische Glas-Metall-Verbindung bildet. Auch hierbei werden verfügbare Kenntnisse und Technologien aus der Glas- und Lichttechnik zur Ausführung der vorliegenden Erfindung sinnvoll genutzt.

Dies gilt auch für eine abgewandelte Ausführung, bei der das zweite Gehäuseteil aus einem Metallring und einem Glas-Mittelteil zusammengesetzt ist, wobei der Umfang des Glas-Mittelteiles mit einer inneren Umfangswand des Metallringes eine physikalisch-chemische Glas-Metall-Verbindung bildet. Die dann noch zu realisierende hermetisch dichte Verbindung zwischen der Außenwand des erwähnten Metallringes und dem ersten Gehäuseteil kann beispielsweise durch ein Laserschweißverfahren geschaffen werden. Derartige Schweißverfahren sind in der Medizintechnik ebenfalls seit langem etabliert und zur Ausführung der Erfindung nutzbar.

Während die vorgenannten Aspekte der Erfindung gewissermaßen eine "High-End-Ausführung" ergeben, ist die Erfindung grundsätzlich auch mit kostengünstigen Kunststoffteilen realisierbar. Hierbei besteht etwa das erste Gehäuseteil aus einem leitfähig gefüllten, insbesondere hochtemperaturbeständigen, Kunststoff und/oder einem Kunststoff mit einer leitfähigen Beschichtung und ist insbesondere als Formblasteil ausgeführt. Im Übrigen kann auch das zweite Gehäuseteil aus Kunststoff bestehen und insbesondere als Spritzgussteil ausgeführt sein. Hiermit kann gewissermaßen eine "Low-Cost-Ausführung" der Erfindung für geringere Ansprüche realisiert werden. Es ist aber auch möglich, Metall- und/oder Glas-Gehäuseteile mit Kunststoff-Gehäuseteilen zu kombinieren.

Während bei einer Ausführung des Tasters mit einem einzelnen Gegenkontakt im zweiten Gehäuseteil das erste Gehäuseteil zugleich als taster-interne Zuleitung zu einem externen Anschlusskontakt dient, kann der Taster auch mit zwei (oder mehr) Gegenkontakten versehen sein, die in das zweite Gehäuseteil eingebettet sind und die durch die in das erste Gehäuseteil integral eingearbeitete Schaltfeder miteinander elektrisch verbunden werden können. In dieser Ausführung überbrückt ein metallischer Abschnitt oder das insgesamt metallisch ausgeführte oder mit Metall beschichtete erste Gehäuseteil nur den Abstand zwischen den Gegenkontakten, ist aber als solches nicht an einen äußeren Anschlusskontakt angeschlossen. Zur Realisierung der Taster-Funktion reicht dann auch eine innenseitige Metallisierung eines ansonsten nicht leitfähigen Kunststoffteils als erstes Gehäuseteil aus.

Je nach Material-Ausführung des vorgeschlagenen Tasters ist der charakteristische thermische Behandlungsschritt zur hermetisch dichten Verbindung der Berührungsstellen der einzelnen Taster-Komponenten entweder als Glas-Metall-Einschmelz- bzw. Sinterschritt, insbesondere bei einer Temperatur oberhalb von 900°C, oder aber als Kunststoff-Warmbearbeitungsschritt ausgestaltet. In letzterer Variante ist es insbesondere ein Schritt des Aufschrumpfens des ersten Gehäuseteils auf das zweite Gehäuseteil oder ein Kunststoffschweiß-Schritt mit an die Verarbeitungseigenschaften des konkret eingesetzten Kunststoffs (Thermoplasten) angepassten Bearbeitungstemperaturen und -zeiten.

Das erfindungsgemäße Handhabungsteil umfasst insbesondere Anschlusskontakte an das erste Gehäuseteil bzw. den oder jeden Gegenkontakt des Tasters, die mit konventionellen Techniken angelötet oder angecrimpt sind. Zur Herstellung der Anschlüsse sind aber auch sonstige Verbindungsverfahren anwendbar, etwa ein Verkleben mit leitfähigem Kleber oder Schweißverfahren, oder auch einfache Steckverbindungen. Speziell können die erwähnten Anschlusskontakte auch Elemente einer SMD-Konfiguration sein, wobei der Taster in Form und Größe dann an die konkrete Konfiguration angepasst ist.

In einer zweckmäßigen Ausgestaltung ist des Weiteren vorgesehen, dass im Inneren des Tasters eine Funktionskomponente der Anordnung platziert ist und deren Anschlüsse hermetisch dicht durch das zweite Gehäuseteil zu Anschlusskontakten im Handhabungsteil geführt sind.

Vorteile und Zweckmäßigkeiten der Erfindung ergeben sich im Übrigen aus der nachfolgenden Beschreibung von Ausführungsbeispielen anhand der Figuren. Von diesen zeigen:
Fig. 1 eine schematische Längsschnittdarstellung eines Tasters gemäß einer ersten Ausführungsform der Erfindung,
Fig. 2 eine schematische Längsschnittdarstellung eines Tasters gemäß einer zweiten Ausführungsform der Erfindung,
Fig. 3 eine schematische Längsschnittdarstellung eines Tasters gemäß einer dritten Ausführungsform der Erfindung,
Fig. 4 eine schematische Längsschnittdarstellung eines Tasters gemäß einer vierten Ausführungsform der Erfindung.

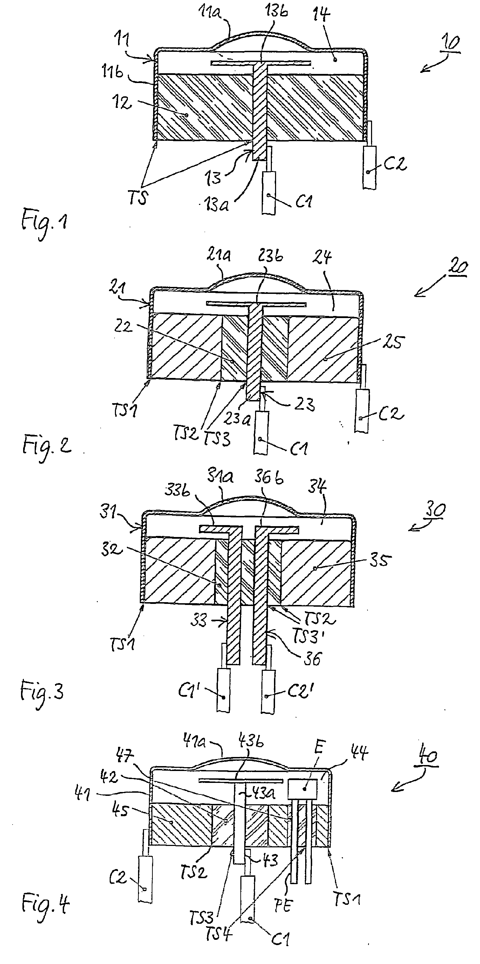

Fig. 1 zeigt einen Taster 10, verbunden mit zwei äußeren Anschlusskontakten C1 und C2, die insbesondere auf einer (nicht dargestellten) Platine eines medizinischen Handhabungsteils angeordnet sein können. Der Taster 10 besteht aus einem ersten Gehäuseteil 11 in Form eines Topfes mit kugelabschnittförmig ausgebeultem Boden 11a und einer zylindrischen Wandung 11b, einem ringförmigen zweiten Gehäuseteil 12 aus Glas und einem in das Glas-Gehäuseteil 12 zentrisch eingesetzten Kontaktstück (Gegenkontakt) 13. Das zweite Gehäuseteil ist in seinem Außendurchmesser so bemessen, dass es praktisch spielfrei in die zylindrische Wandung 11b des ersten Gehäuseteils 11 passt, und in die zylindrische Durchführung in seinem Zentrum fast ebenso spielfrei ein Stiftabschnitt 13a des Kontaktstücks 13.

Durch einen thermischen Bearbeitungsschritt (siehe weiter unten) ist an den Berührungsflächen der genannten Teile jeweils eine hermetisch dichte Versiegelung TS geschaffen. Das Kontaktstück 13 ist im Taster 10 so platziert, dass eine kreisförmige Kontaktplatte 13b an seinem inneren Ende in einem Hohlraum 14 zwischen der innenseitigen Stirnfläche des zweiten Gehäuseteils 12 und dem Boden des ersten Gehäuseteils 11 zu liegen kommt, und zwar mit solchem Abstand unterhalb der Beule 11a im Boden des ersten Gehäuseteils, das diese beim federnden Niederdrücken die Kontaktplatte 13b berührt. Hierdurch wird eine (temporäre) elektrische Verbindung zwischen dem Anschlusskontakt C1, an den das Kontaktstück 13 angeschlossen ist, und dem Anschlusskontakt C2 hergestellt, an den das leitfähige erste Gehäuseteil 11 angeschlossen ist. Die Beule 11a dient somit als Schaltfeder des Tasters 10.

Ein in Fig. 2 dargestellter modifizierter Taster 20 hat weitgehend den gleichen Aufbau wie der Taster 10 aus Fig. 1, und insoweit sind seine Teile mit korrespondierenden Bezugsziffern bezeichnet und werden hier nicht nochmals erläutert. Abweichend von der ersten Ausführungsform ist der Aufbau des zweiten Gehäuseteils aus einem Glas-Innenring 22, welcher das Kontaktstück 23 hält und führt, und einem diesen umgebenden Metall-Außenring 25, der sich bis zur Innenwand des ersten Gehäuseteils 21 erstreckt. Durch geeignete Dimensionierung und thermische Behandlung sind bei dieser Ausführung drei (bis 2,5 bar) hermetisch dichte Versiegelungsbereiche TS1 (zwischen erstem Gehäuseteil 21 und Außenring 25), TS2 (zwischen Innenring 22 und Außenring 25) und TS3 (zwischen Innenring 22 und Kontaktstück 23) ausgebildet.

Fig. 3 zeigt einen weiteren Taster 30 als gegenüber Fig. 2 weiter modifizierte Ausführung. Auch hier sind wieder mit der Ausführung nach Fig. 2 übereinstimmende Teile mit zu Fig. 2 korrespondierenden Bezugsziffern bezeichnet und werden hier nicht weiter erläutert. Die wesentlichen Unterschiede bestehen in einer anderen Anordnung der äußeren Anschlusskontakte C1' und C2' und dem hierauf abgestimmten Vorsehen zweier Kontaktstücke (Gegenkontakte) 33 und 36, deren Kontaktplatten 33b bzw. 36b jeweils unterhalb der Schaltfeder 31a des ersten Gehäuseteils 31 im Innenraum 34 des Tasters liegen. Durch das Niederdrücken der Schaltfeder 31a kommt diese mit beiden Kontaktplatten 33b, 36b gleichzeitig in Kontakt und stellt hierdurch eine elektrische Verbindung zwischen den Kontaktstücken 33 und 36 und somit zwischen den äußeren Anschlusskontakten C1' und C2' her.

Fig. 4 zeigt als weitere Ausführungsform der Erfindung einen Taster 40 mit einer Zusatzfunktion, der ansonsten im Wesentlichen dem Taster 20 aus Fig. 2 entspricht. Der Innenraum 44 des Tasters 40 ist hier etwas größer dimensioniert, und zwar indem die Dicke der gemeinsam das zweite Gehäuseteil bildenden Ringe 42 und 45 gegenüber der zweiten Ausführungsform reduziert ist. Hierdurch wird Platz für ein elektronisches Bauelement E geschaffen, welches geschützt im Innenraum untergebracht ist und dessen Anschlussstifte PE hermetisch dicht durch das zweite Gehäuseteil geführt sind. Der Metall-Außenring 45 weist hierzu eine Bohrung auf, in die ein Glasstopfen 47 eingesetzt ist, in den zuvor die Anschlussstifte PE des Bauelements E eingeschmolzen wurden.

Die Ausführung der für die Versiegelungen erforderlichen Hochtemperaturschritte erfolgt in einem Ofen bei einer Temperatur im Bereich zwischen 900 und 1000°C, bei der das für die Glasteile der jeweiligen Taster verwendete Glas eine physikalisch-chemische Glas-Metall-Verbindung mit den jeweils angrenzenden Metallteilen (die insbesondere aus Edelstahl bestehen) eingeht. Die Taster werden hierzu auf eine Graphitplatte verbracht und in den Ofen gefahren und dort für eine vorbestimmte Zeit bei der erforderlichen Temperatur gehalten. Das Einschmelzen der Kontaktstücke bzw. (bei der Ausführung nach Fig. 4) auch der Kontaktstifte des Bauelements in das jeweils zugehörige Glasteil kann vorab in einem separaten thermischen Prozess geschehen sein. Der Anschluss des fertigen Tasters an die äußeren Anschlusskontakte erfolgt mit einem herkömmlichen Löt- oder Schweißverfahren, es kommen aber auch ein formschlüssiges Verbindungsverfahren wie das Crimpen oder eine Steckverbindung in Betracht, wenn die Verbindungsabschnitte geeignet konfiguriert sind (etwa bei der Ausführung nach Fig. 3).

## Patentansprüche

1. Taster (10; 20; 30; 40), insbesondere eines medizinischen Handhabungsteiles, welcher aufweist:
ein erstes, topf-, schalen- oder kastenartiges Gehäuseteil (11; 21; 31; 41), mindestens abschnittsweise mit leitfähigem Material, mit einer integral eingeformten Schaltfeder (11a; 21a; 31a; 41a) und einer zusammenhängenden Wandung,
ein zweites Gehäuseteil (12; 22; 25; 32; 35; 42; 45), das in seiner Form an die Wandung des ersten Gehäuseteils angepasst und in dieses hermetisch dicht derart eingefügt ist, dass zwischen dem ersten und zweiten Gehäuseteil unter der Schaltfeder ein Hohlraum gebildet ist, und
mindestens einen hermetisch dicht und gegenüber dem ersten Gehäuseteil elektrisch isoliert durch das zweite Gehäuseteil geführten und in den Hohlraum unterhalb der Schaltfeder ragenden Schaltfeder-Gegenkontakt (13; 23; 33; 36; 43),**dadurch gekennzeichnet, dass**
das erste Gehäuseteil aus federelastischem Metall, insbesondere Edelstahl oder Titan oder einer Titanlegierung, besteht und insbesondere als Tiefziehteil ausgeführt ist und
das zweite Gehäuseteil vollständig aus Glas besteht (12), der Gegenkontakt in den Glasabschnitt eingeschmolzen ist und der die Wand des ersten Gehäuseteils (11) berührende Umfang des zweiten Gehäuseteils mit jener Wand eine physikalisch-chemische Glas-Metall-Verbindung bildet, oder dass das zweite Gehäuseteil aus einem Metallring (25; 35; 45) und einem Glas-Mittelteil (22; 32; 42) zusammengesetzt ist und der Umfang des Glas-Mittelteils mit einer inneren Umfangswand des Metallringes eine physikalisch-chemische Glas-Metall-Verbindung bildet.

2. Taster (30) nach Anspruch 1, mit zwei Gegenkontakten (33; 36), deren in den Hohlraum ragende Enden derart positioniert sind, dass die Schaltfeder (31a) sie beim Betätigen gemeinsam berührt.

3. Verfahren zur Herstellung eines Tasters (10; 20; 30; 40)nach einem der vorangehenden Ansprüche, mit einem thermischen Behandlungsschritt zur hermetisch dichten Verbindung zwischen der Wand des ersten Gehäuseteils (11; 21; 31; 41) und dem Umfang des zweiten Gehäuseteils (12; 22; 25; 32; 35; 42; 45) und/oder zwischen dem oder jedem Gegenkontakt (13; 23; 33; 36;43) und der Wand der dafür vorgesehenen Durchführung im zweiten Gehäuseteil.

4. Verfahren nach Anspruch 3, wobei der thermische Behandlungsschritt als Glas-Metall-Einschmelz- bzw. Sinterschritt, insbesondere oberhalb von 900°C, ausgeführt wird.

5. Medizinisches Handhabungsteil, insbesondere Instrument oder Handgriff, mit einem Taster (10; 20; 30; 40) nach Anspruch 1 oder 2, insbesondere ohne zusätzliche Dichtmittel, und zwei Anschlusskontakten (C1; C2; C1'; C2'), die an das erste Gehäuseteil (11; 21 ; 31; 41) und den Gegenkontakt (13; 23; 43) bzw. an zwei Gegenkontakte (33; 36) des Tasters angeschlossen sind.

6. Handhabungsteil nach Anspruch 5, wobei die Anschlusskontakte (C1;C2; C1'; C2') an das erste Gehäuseteil (11; 21; 31; 41) bzw. den oder jeden Gegenkontakt (13; 23; 33; 36; 43) des Tasters angelötet oder angecrimpt sind.

7. Handhabungsteil nach Anspruch 5 oder 6, wobei die Anschlusskontakte (C1; C2; C1'; C2') Elemente einer SMD-Konfiguration sind und der Taster (10; 20; 30; 40) in Form und Größe hieran angepasst ist.

8. Handhabungsteil nach einem der Ansprüche 5 bis 7, wobei im Inneren des Tasters (40) eine Funktionskomponente (E) des Handhabungsteils platziert ist und deren Anschlüsse (PE) hermetisch dicht durch das zweite Gehäuseteil (42)zu Anschlusskontakten im Handhabungsteil geführt sind.

## Claims

1. Button (10; 20; 30; 40), in particular of a medical handling part which has:
a first pot-shaped, shell-shaped or box-shaped housing part (11; 21; 31; 41) having conductive material at least in sections, having an integrally moulded switching spring (11a; 21a; 31a; 41a) and a continuous wall,
a second housing part (12; 22; 25; 32; 35; 42; 45), the shape of which is adapted to the wall of the first housing part and is inserted into this in a hermetically sealed manner such that a cavity is formed between the first and second housing part underneath the switching spring and
at least one switching spring counter contact (13; 23; 33; 36; 43) which is guided, hermetically sealed and electrically insulated from the first housing part, through the second housing part and protrudes into the cavity underneath the switching spring,
**characterised in that**
the first housing part consists of resilient metal, in particular stainless steel or titanium or a titanium alloy and, in particular, is implemented as a deep-drawn part and
the second housing part consists entirely of glass (12), the counter contact is melt-sealed into the glass section and the circumference of the second housing part touching the wall of the first housing part (11) forms a physicochemical glass-metal connection with each wall, or the second housing part is made up of a metal ring (25; 35; 45) and a glass middle part (22; 32; 42) and the circumference of the glass middle part forms a physicochemical glass-metal connection with an inner circumferential wall of the metal ring.

2. Button (30) according to claim 1, having two counter contacts (33; 36) whose ends protruding into the cavity are positioned in such a way that the switching spring (31a) touches them mutually during actuation.

3. Method for the production of a button (10; 20; 30; 40) according to one of the preceding claims, having a thermal treatment step for the hermetically sealed connection between the wall of the first housing part (11; 21; 31; 41) and the circumference of the second housing part (12; 22; 25; 32; 35; 42; 45) and/or between the or each counter contact (13; 23; 33; 36; 43) and the wall of the feed-through provided therefor in the second housing part.

4. Method according to claim 3, wherein the thermal treatment step is implemented as a glass-to-metal melt-sealing or sintering step, in particular above 900°C.

5. Medical handling part, in particular instrument or handle, having a button (10; 20; 30; 40) according to claim 1 or 2, in particular without additional sealing means, and two connection contacts (C1; C2; C1'; C2') which are connected to the first housing part (11; 21; 31; 41) and the counter contact (13; 23; 43) or to two counter contacts (33; 36) of the button.

6. Handling part according to claim 5, wherein the connection contacts (C1; C2; C1'; C2') are soldered or crimped onto the first housing part (11; 21; 31; 41) or onto the or each counter contact (13; 23; 33; 36; 43) of the button.

7. Handling part according to claim 5 or 6, wherein the connection contacts (C1; C2; C1'; C2') are elements of an SMD configuration and the shape and size of the button (10; 20; 30; 40) are adapted thereto.

8. Handling part according to one of claims 5 to 7, wherein a functional component (E) of the handling part is placed in the interior of the button (40) and the connections (PE) thereof are guided in a hermetically sealed manner through the second housing part (42) to connection contacts in the handling part.

## Revendications

1. Bouton-poussoir (10; 20; 30; 40), en particulier d'un élément de manipulation médical, qui présente :
un premier élément de boîtier (11; 21; 31; 41) en forme de pot, de coupe ou de caisson, au moins en partie avec un matériau conducteur, avec un ressort d'enclenchement (11a; 21a; 31a; 41a) formé d'une seule pièce, et une paroi continue,
un deuxième élément de boîtier (12; 22; 25; 32; 35; 42; 45) dont la forme est adaptée à la paroi du premier élément de boîtier et qui est inséré hermétiquement dans celui-ci de manière telle qu'une cavité soit formée entre le premier et le deuxième élément de boîtier, sous le ressort d'enclenchement, et
au moins un contre-contact (13; 23; 33; 36; 43) de ressort d'enclenchement qui est guidé à travers le deuxième élément de boîtier de façon hermétique et en étant isolé électriquement vis-à-vis du premier élément de boîtier et qui avance dans la cavité située sous le ressort d'enclenchement, **caractérisé en ce que**
le premier élément de boîtier est constitué d'un métal ayant l'élasticité d'un ressort, notamment d'acier inoxydable ou de titane ou d'un alliage de titane, et est réalisé notamment comme pièce emboutie, et
le deuxième élément de boîtier est constitué entièrement de verre (12), le contre-contact est scellé dans la partie en verre, et le pourtour du deuxième élément de boîtier qui est en contact avec la paroi du premier élément de boîtier (11) forme avec cette paroi une liaison verre-métal physico-chimique, ou **en ce que** le deuxième élément de boîtier est assemblé à partir d'une bague métallique (25; 35; 45) et d'un élément central en verre (22; 32; 42) et le pourtour de l'élément central en verre forme une liaison verre-métal physico-chimique avec une paroi périphérique intérieure de la bague métallique.

2. Bouton-poussoir (30) selon la revendication 1, comprenant deux contre-contacts (33; 36) dont les extrémités qui avancent dans la cavité sont positionnées de manière telle que le ressort d'enclenchement (31a) soit en contact commun avec elles lors de l'actionnement.

3. Procédé de fabrication d'un bouton-poussoir (10; 20; 30; 40) selon l'une des revendications précédentes, comprenant une étape de traitement thermique pour la liaison hermétique entre la paroi du premier élément de boîtier (11; 21; 31; 41) et le pourtour du deuxième élément de boîtier (12; 22; 25; 32; 35; 42; 45) et/ou entre le contre-contact (13; 23; 33; 36; 43) ou chaque contre-contact et la paroi du passage prévu à cet effet dans le deuxième élément de boîtier.

4. Procédé selon la revendication 3, selon lequel l'étape de traitement thermique est exécutée en tant qu'étape de scellement verre-métal ou de frittage, en particulier au-dessus de 900 °C.

5. Elément de manipulation médical, en particulier instrument ou poignée, comprenant un bouton-poussoir (10; 20; 30; 40) selon la revendication 1 ou 2, en particulier sans moyens d'étanchéité supplémentaires, et deux contacts de connexion (C1; C2; C1'; C2') qui sont connectés au premier élément de boîtier (11; 21; 31; 41) et au contre-contact (13; 23; 43) ou à deux contre-contacts (33, 36) du bouton-poussoir.

6. Elément de manipulation selon la revendication 5, où les contacts de connexion (C1; C2; C1'; C2') sont brasés ou sertis sur le premier élément de boîtier (11; 21; 31; 41) ou sur le ou sur chaque contre-contact (13; 23; 33; 36; 43) du bouton-poussoir.

7. Elément de manipulation selon la revendication 5 ou 6, où les contacts de connexion (C1; C2; C1'; C2') sont des éléments d'une configuration CMS, et la forme et la taille du bouton-poussoir (10; 20; 30; 40) sont adaptées à celle-ci.

8. Elément de manipulation selon l'une des revendications 5 à 7, où un composant fonctionnel (E) de l'élément de manipulation est placé à l'intérieur du bouton-poussoir (40), et les bornes (PE) de celui-ci sont guidées de façon hermétique à travers le deuxième élément de boîtier (42), jusqu'à des contacts de connexion dans l'élément de manipulation.
